**Europäisches Patentamt**

(19) **European Patent Office**

**Office européen des brevets**

(11) Veröffentlichungsnummer: **0 145 938**
**B1**

(12) **EUROPÄISCHE PATENTSCHRIFT**

(45) Veröffentlichungstag der Patentschrift:
18.05.88

(51) Int. Cl.⁴: **A 61 F 2/36**

(21) Anmeldenummer: **84113454.7**

(22) Anmeldetag: **06.11.84**

(54) In einen Röhrenknochen einsetzbarer Prothesenschaft.

(30) Priorität: **30.11.83 DE 3343304**

(43) Veröffentlichungstag der Anmeldung:
**26.06.85 Patentblatt 85/26**

(45) Bekanntmachung des Hinweises auf die Patenterteilung:
**18.05.88 Patentblatt 88/20**

(84) Benannte Vertragsstaaten:
**AT BE CH DE FR GB IT LI NL SE**

(56) Entgegenhaltungen:
**EP-A-0 027 160**
**EP-A-0 050 533**
**FR-A-2 483 218**

(73) Patentinhaber: **Protek AG, Stadtbachstrasse 64, CH-3012 Bern (CH)**

(72) Erfinder: **Weill, Dan, Dr., Château de buy Antilly, F-57640 Vigy (FR)**

(74) Vertreter: **Lusuardi, Werther G., Dr. Lusuardi AG Stockerstrasse 8, CH- 8002 Zürich (CH)**

## Beschreibung

Die Erfindung betrifft einen in einen Röhrenknochen einsetzbaren Prothesenschaft zur zementfreien Verankerung einer Endoprothese mit einer anterioren, einer posterioren, einer medialen und einer lateralen Schaftfläche, dessen distales Ende durch einen von distal nach proximal und von anterior nach posterior verlaufenden Schlitz der Länge L in einen lateralen und einen medialen Schenkel gespalten ist. Prothesenschäfte der vorstehend genannten Art zur zementfreien Verankerung in einem Röhrenknochen, beispielsweise Verankerungsschäfte für eine Femurkopfprothese, sind z. B.aus der EP-A1-0 050 533 bekannt.

Bei diesem bekannten Verankerungsschaft liegt der Längsschlitz in der Symmetrieebene der Prothese. Damit sind die physikalischen Eigenschaften der lateralen und medialen Schaftspitze identisch. Bei grossen Schäften ergibt sich somit eine insgesamt starre Schaftspitze (d. h. der Längsschlitz übt im wesentlichen keine Funktion aus), während es bei kleinen Schäften zu einer beidseitigen, lateralen und medialen Verformung der Schaftspitze kommt. Bei einer Verformung des lateralen Teils der Schaftspitze geht aber die Stabilität des implantierten Schaftes verloren.

Aufgabe der Erfindung ist es, einen Prothesenschaft der eingangs erwähnten Art für die Verankerung, insbesondere einer Femurkopfprothese, bereitzustellen, mit dem eine gewisse Anpassung an die individuell unterschiedlichen Markhohlräumen im distalen Bereich des Schaftes ohne Gefährdung der Implantatstabilität möglich ist und dadurch auch mit einigen wenigen Schaftgrössen alle Anforderungen erfüllt werden können.

Die Lösung dieser Aufgabe erfolgt nach der vorliegenden Erfindung dadurch, dass der Schlitz bezüglich der Mittelachse der distalen Hälfte des Schaftes so weit nach medial verschoben ist, dass der mediale Schenkel plastisch und elastisch verformbar ist und der laterale Schenkel im wesentlichen starr ist.

Durch die erfindungsgemässe Massnahme wird der mediale Teil der Schaftspitze in seinen Dimensionen so verringert, dass er plastisch und elastisch verformbar wird, während der laterale Teil der Schaftspitze durch eine entsprechende Verstärkung des Querschnitts praktisch starr bleibt und eine stabile Implantation garantiert.

Die Schaftspitze ist dadurch insgesamt in Richtung von medial nach lateral, also parallel zu den Blattseiten zusammendrückbar und kann sich damit in gewissem Umfang an unterschiedliche Hohlräume anpassen, wobei eine plastische Verformung gegebenenfalls bereits vom Operateur vor dem Einsetzen des Schaftes vorgenommen werden kann.

Vorteilhafterweise wird der Längsschlitz am distalen Schaftende so ausgebildet, dass die Breite (A) des medialen Verformungsquerschnittes sich zur Länge (L) des Schlitzes wie 1 : 4 bis 1 : 10, vorzugsweise wie 1 : 4,5 bis 1 : 5,5 verhält, wobei die Länge des Längsschlitzes etwa 25 - 50 mm beträgt. Zweckmässigerweise sollte der Längsschlitz parallel zur Mittelachse verlaufen.

Vorzugsweise ist der Prothesenschaft als Geradschaft ausgebildet, welcher sich mindestens in der distalen Hälfte seiner, entlang der Mittelachse gemessenen Schafthöhe mindestens einseitig konisch erweitert.

Als Werkstoffe für den neuen Schaft eignen sich alle in der Implantat-Technik üblichen Materialien, die die notwendige Festigkeit, Elastizität und Plastizität haben; in erster Linie werden die neuen Schäfte daher aus Metall oder Metall-Legierungen verfertigt.

Im folgenden wird die Erfindung anhand eines Ausführungsbeispiels im Zusammenhang mit der Zeichnung näher erläutert.

Fig. 1 zeigt eine Aufsicht auf die neue Prothese von anterior oder posterior;

Fig. 2 und 3 geben als Detailvariante von Fig. 1 das distale Ende des neuen Schaftes wieder, wobei Fig. 3 eine Ansicht von Fig. 2 von rechts ist.

Die laterale Schmalseite 4 des Schaftblattes 1 des neuen Prothesenschaftes erweitert sich von ihrem distalen Ende 9 her konisch bis etwa auf 2/3 der längs der Mittelachse 2 gemessenen Schafthöhe; vom proximalen Ende des Konus führt die laterale Schmalseite 4 nach medial gekrümmt in eine horizontale Schulter am proximalen Ende des Schaftes. Diese Schulter geht in einen Prothesenhals 6 über, auf den ein sich nach aussen konisch verjüngender Zapfen 7 aufgesetzt ist, der den nicht gezeigten kugelförmigen Gelenkkopf aufnimmt.

Die Achse 8 des Zapfens 7 schneidet die Längsmittelachse 2 des Schaftes unter einem Winkel, der im wesentlichen dem Winkel zwischen dem Schenkelhals und der Femurachse eines natürlichen Hüftgelenks entspricht und im vorliegenden Fall etwa 45° beträgt.

Die mediale Schmalseite 3 verläuft zunächst parallel zur Mittelachse 2 und geht anschliessend in einen Bogen über, der in seinem Verlauf weitgehend dem Calcar-Bogen angepasst ist.

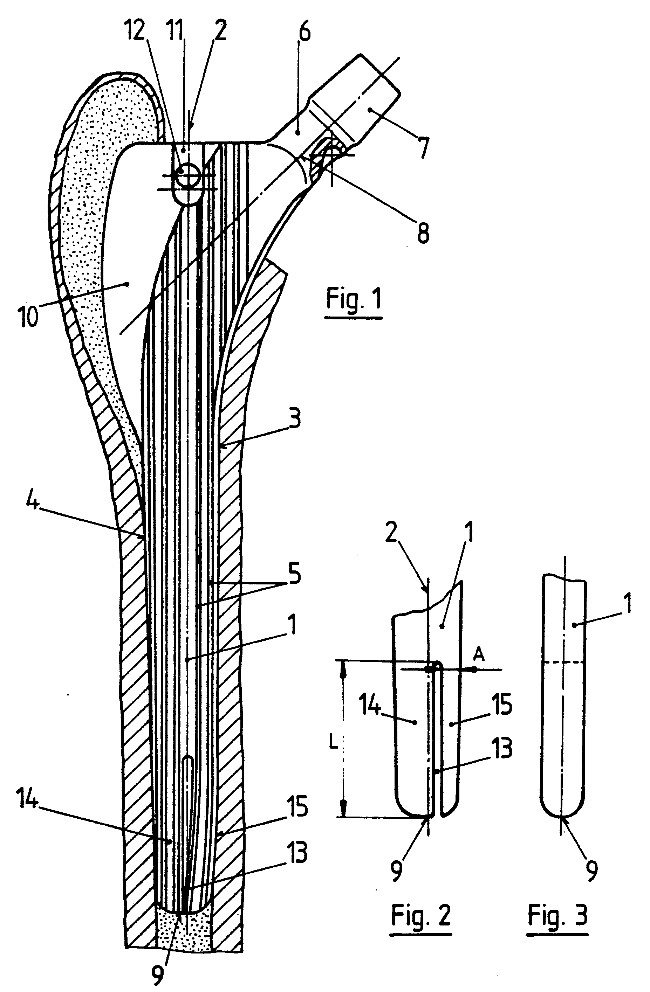

Die Blattseiten des Schaftblattes 1, die, wie Fig. 3 wiedergibt, über die ganze Schafthöhe parallel zueinander sind, sind mit einer aus sich parallel zur Mittelachse 2 erstreckenden Rillen 5 bestehenden Oberflächenstruktur versehen, die das Anwachsen von Knochengewebe fördert.

Aus der lateralen Schmalseite 4 des Schaftblattes 1 springt - etwa zwischen halber und 3/5 der Schafthöhe - in einem zunächst nach lateral gekrümmten Bogen ein Trochanterflügel 10 hervor, der, sich nach proximal verbreiternd, im wesentlichen der Krümmung der lateralen Schmalseite 4 folgt und ebenfalls in der horizontalen Schulter endet. In ihm befinden sich Ausnehmungen 11 und eine Bohrung 12 für den Einsatz eines Setz- und Führungsinstruments bzw. eines Ausziehinstrumentes.

Parallel zur Mittelachse 2 ist am distalen Ende 9 des Schaftblattes 1 erfindungsgemäss ein Längsschlitz 13 vorgesehen, der aus der Mittelachse 2 heraus nach medial versetzt ist, so dass die Breite A des Verformungsquerschnitts, die zur Länge L des Schlitzes 13 in einem Verhältnis von 1 : 5 steht, auf der medialen Seite, d. h. am Schenkel 15 kleiner ist als diejenige des lateralen Schenkels 14. Hierdurch werden die Verformungseigenschaften so verändert, dass der Schenkel 14 praktisch starr, also- durch die auftretenden Belastungen- unverformbar ist, und die ganze Verformung im medialen Schenkel 15 erfolgt. Diese Massnahme hat den Sinn, bei eingesetzter Prothese den von lateral nach distal zur Auflage des Schaftes auf dem Calcar-Bogen ausgeübten "Druck" als Gegenkraft gegen eine Biegebelastung aufgrund des vom Gelenkkopf ausgeübten Momentes zu nutzen, was nur bei einer starren, lateralen Schaftspitze möglich ist.

## Patentansprüche

1. In einen Röhrenknochen einsetzbarer Prothesenschaft zur zementfreien Verankerung einer Endoprothese mit einer anterioren, einer posterioren, einer medialen und einer lateralen Schaftfläche, dessen distales Ende (9) durch einen von distal nach proximal und von anterior nach posterior verlaufenden Schlitz (13) der Länge L in einen lateralen (14) und einen medialen (15) Schenkel gespalten ist, dadurch gekennzeichnet, dass der Schlitz (13) bezüglich der Mittelachse (2) der distalen Hälfte des Schaftes soweit nach medial verschoben ist, dass der mediale Schenkel (15) plastisch und elastisch verformbar ist und der laterale Schenkel (14) im wesentlichen starr ist.

2. Prothesenschaft nach Anspruch 1, dadurch gekennzeichnet, dass der mediale Schenkel (15) eine geringere Biegefestigkeit aufweist als der laterale Schenkel (14).

3. Prothesenschaft nach Anspruch 1 oder 2, dadurch gekennzeichnet, dass die in mediolateraler Richtung gemessene Breite A des medialen Schenkels (15) sich zur Länge L des Schlitzes (13) verhält wie 1 : 4 bis 1 : 10, vorzugsweise wie 1 : 4,5 bis 1 : 5,5.

4. Prothesenschaft nach einem der Ansprüche 1 bis 3, dadurch gekennzeichnet, dass er als Geradschaft ausgebildet ist.

5. Prothesenschaft nach einem der Ansprüche 1 bis 4, dadurch gekennzeichnet, dass er sich mindestens in der distalen Hälfte seiner, entlang der Mittelachse (2) gemessenen Schafthöhe von distal nach proximal mindestens einseitig erweitert.

6. Prothesenschaft nach einem der Ansprüche 1 bis 5, dadurch gekennzeichnet, dass die anteriore und posteriore Schaftfläche parallel zueinander verlaufen.

7. Prothesenschaft nach einem der Ansprüche 1 bis 6, dadurch gekennzeichnet, dass der Schlitz (13) parallel zur Mittelachse (2) verläuft.

## Claims

1. Prosthesis stem for cement-free anchorage of an endo-prosthesis into a tubular bone with an anterior, a posterior, a medial and a lateral stem surface, the distal end (9) of said stem being split into a lateral (14) and a medial (15) leg by a slit (13) of the length L running from the distal to the proximal side and from the anterior to the posterior side, characterized in that said slit (13) is displaced medially with regard to the center axis (2) of the distal half of said stem by such an amount that said medial leg (15) is plastically and elastically deformable and said lateral leg (14) is essentially rigid.

2. Prosthesis stem according to claim 1, characterized in that said medial leg (15) has a lower bending strength than said lateral leg (14).

3. Prosthesis stem according to claim 1 or 2, characterized in that the ratio that the width A of said medial leg (15) measured in medio-lateral direction bears to said length L of said slit (13) is between 1 : 4 and 1 : 10, preferably between 1 : 4.5 and 1 : 5.5.

4. Prosthesis stem according to one of the claims 1 to 3, characterized in that it is designed as a straight stem.

5. Prosthesis stem according to one of the claims 1 to 4, characterized in that it widens at least in the distal half of its stem hight measured along the center axis (2) from distal to proximal at least on one side of said axis (2).

6. Prosthesis stem according to one of the claims 1 to 5, characterized in that said anterior and posterior stem surfaces are parallel to each other.

7. Prosthesis stem according to one of the claims 1 to 6, characterized in that said slit (13) is parallel to said center axis (2).

## Revendications

1. Tige de prothèse pour fixation sans ciment d'une endoprothèse dans un os tubulaire avec une surface de tige antérieure, postérieure, médiale et latérale, l'extrémité distale (9) de ladite tige étant divisée par une fente (13) de la longeur L s'étendant du côté distal au côté proximal et du côté antérieur au côté postérieur en une branche latérale (14) et une branche médiale (15), caractérisée en ce que ladite fente (13) est déplacée en direction médiale par rapport à l'axe central (2) de la moitié distale de ladite tige de façon à rendre ladite branche médiale (15) plastiquement et élastiquement déformable et ladite branche latérale (14) essentiellement rigide.

2. Tige de prothèse selon la revendication 1, caractérisée en ce que ladite branche médiale (15) présente une résistance à la flexion inférieure à celle de ladite branche latérale (14).

3. Tige de prothèse selon la revendication 1 ou 2, caractérisée en ce que la largeur A de ladite branche médiale (15) mesurée en direction médio-latérale se rapporte à la longeur L de ladite fente (13) comme 1 : 4 jusqu'à 1 : 10, préférablement comme 1 : 4,5 jusqu'à 1 : 5,5.

4. Tige de prothèse selon une des revendications 1 à 3, caractérisée en ce qu'elle est en forme de tige droite.

5. Tige de prothèse selon une des revendications 1 à 4, caractérisée en ce qu'elle s'élarge au moins dans la moitié distale de son hauteur de tige mesurée le long de ledit axe central (2) du côté distal au côté proximal au moins d'un côté de ledit axe (2).

6. Tige de prothèse selon une des revendications 1 à 5, caractérisée en ce que lesdites surfaces de tige antérieures et postérieures sont parallèles.

7. Tige de prothèse selon une des revendications 1 à 6, caractérisée en ce que ladite fente (13) est parallèle avec ledit axe central (2).

Fig. 1

Fig. 2

Fig. 3